(19) Europäisches Patentamt
European Patent Office
Office européen des brevets

(11) Veröffentlichungsnummer: **0 361 301 B1**

(12) # EUROPÄISCHE PATENTSCHRIFT

(45) Veröffentlichungstag der Patentschrift: **03.03.93**

(51) Int. Cl.5: **A01N 47/44**, A61K 6/10, A61L 2/18, //(A01N47/44,33:12)

(21) Anmeldenummer: **89117396.5**

(22) Anmeldetag: **20.09.89**

Die Akte enthält technische Angaben, die nach dem Eingang der Anmeldung eingereicht wurden und die nicht in dieser Patentschrift enthalten sind.

(54) **Mikrobizide Zubereitungen für die Desinfektion von Dentalformkörpern.**

(30) Priorität: **22.09.88 DE 3832161**

(43) Veröffentlichungstag der Anmeldung:
**04.04.90 Patentblatt 90/14**

(45) Bekanntmachung des Hinweises auf die Patenterteilung:
**03.03.93 Patentblatt 93/09**

(84) Benannte Vertragsstaaten:
**AT CH DE ES FR GB IT LI**

(56) Entgegenhaltungen:
**EP-A- 0 185 970**
**EP-A- 0 252 310**
**EP-A- 0 265 776**

(73) Patentinhaber: **Merz & Co. GmbH & Co.**
**Eckenheimer Landstrasse 100-104**
**W-6000 Frankfurt/M. 1(DE)**

(72) Erfinder: **Thamm, Rüdiger, Dr. Dipl.-Chem.**
**Bornweg 6**
**W-6101 Messel(DE)**

(74) Vertreter: **Beil, Hans Christoph, Dr. et al**
**Beil, Wolff und Beil Rechtsanwälte Adelonstrasse 58**
**W-6230 Frankfurt am Main 80 (DE)**

EP 0 361 301 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung mikrobizider Zubereitungen zur Desinfektion von Dentalformkörpern und Abformmaterialien.

Trotz bekannter Gefahren gibt es heute nach wie vor aus den verschiedensten Gründen erhebliche Hygienedefizite in der zahnmedizinischen Praxis. Ein dort besonders aktuelles und ungelöstes Problem ist die Desinfektion prothetischer Dentalformstücke und Dentalformmassen, deren Kontamination mit pathogenen Keimen durch beispielsweise anhaftende Speichel-und Blutreste als permanent gegeben anzusehen ist. Die Notwendigkeit einer Desinfektion dieser Dentalmaterialien im Sinne einer Infektionsprophylaxe sollte sich hieraus zwanglos ergeben. Wegen der Vielfalt der Materialien und deren unterschiedlicher Empfindlichkeit gegenüber Einflüssen chemischer Desinfektionsverfahren ist jedoch derzeit kein praktikables Verfahren zur Dekontamierung von Dentalwerkstoffen bekannt (Dental Magazin, 4, Nr. 4, 90 (1986), Dental-Echo 1, 87, 52 (1987, Zahnärztliche Praxis 7, 265 (1988)).

EP-A-0 265 776 (1988) beschreibt ein Verfahren zur Desinfektion eines Dentalformmateriales aus Alginate, das aus einer Mischung aus Alginate, Wasser und einer mikrobioziden Substanz besteht. Das Biozid enthält quartäre Ammoniumverbindungen (z.B. Didecyldimethylammonium chlorid), Bisguanidinverbindungen, Quinolinverbindungen, substituierte Phenole und Mischungen davon.

Neben ökonomischen, ökologischen, toxikologischen u.a. Aspekten sind an ein chemisches Mittel zur desinfektorischen Anwendung bei Dentalwerkstoffen in der zahnärztlichen Praxis folgende Anforderungen zu stellen: breites mikrobizides Wirkungsspektrum, schneller Wirkungseintritt, hohe Keimzahl-Reduktion, keine Dimensions- und/oder Oberflächenveränderungen der Werkstoffe sowie der Gipsmodelle, problemlose Anwendung, keine Beeinträchtigung der Arbeitsabläufe, keine Beeinflussung des Faktors Zeit, universelle Anwendbarkeit. Unter toxikologischen und ökologischen Gesichtspunkten bestehen dabei insbesondere Bedenken gegen Desinfektionsmittel mit Aldehyd-Gehalt.

EP-A-0 252 310 beschreibt formaldehydfreie antimikrobielle Mittel zur Desinfektion von harten und weichen Oberflächen im medizinischen Bereich, die sich von dem erfindungsgemäßen Mittel dadurch unterscheiden, daß unsubstituierte Benzylammoniumsalze zusätzlich eigesetzt werden.

Überraschend wurde gefunden, dass bestimmte aldehydfreie mikrobiziden Zubereitungen die vorstehenden Forderungen sämtlich erfüllen und deshalb zur Desinfektion von Dental-Formkörpern, insbesondere solcher auf Silikon-, Acrylat-, Polyether-, Alginat-, Gips- und Hydrokolloidbasis, ausgezeichnet geeignet sind.

Weiter wurde überraschend jedoch gefunden, dass bei Einsatz der erfindungsgemässen Zubereitungen bei der Herstellung von Gips- und Alginat-Abdruckmassen nicht nur die Formmasse als solche desinfiziert wird, sondern der daraus hergestellte Formkörper desinfizierend gegenüber einer äusserlichen Kontamination wirkt. Dazu ist es nur erforderlich, dass die nach der Herstellerangabe für die Zubereitung des Abformmaterials vorgesehene Wassermenge durch die gleiche Menge der erfindungsgemässen Zubereitung ersetzt wird.

Die erfindungsgemäss zu verwendenden mikrobiziden Zubereitungen enthalten quartäre Ammoniumsalze der allgemeinen Formel

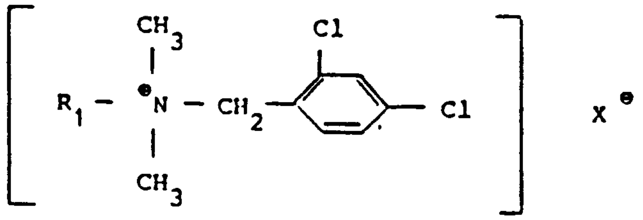

(I)

und

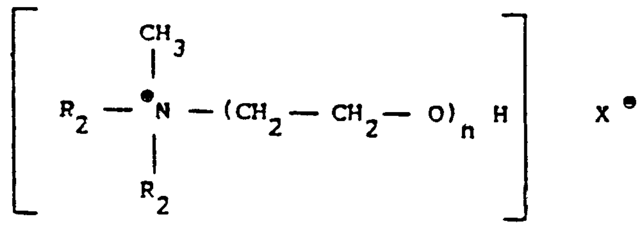

(II)

worin $R_1$ einen Alkylrest mit 8 bis 18 Kohlentoffatomen, $R_2$ einen Alkylrest mit geradzahliger Kohlenwasserstoffkette von 8 bis 16 Kohlenstoffatomen, n ganze Zahl von 1 bis 5 einschliesslich und $X^-$ das Anion einer anorganischen und/oder organischen Säure bedeuten, und eine Diguanidin-Verbindung der allgemeinen Formel

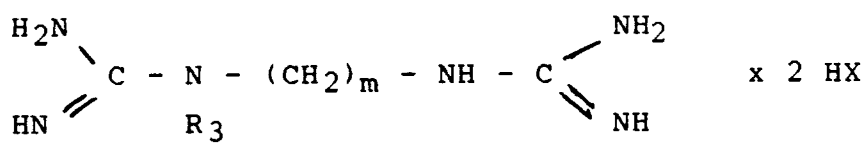

(III)

worin $R_3$ einen Alkylrest mit 8 bis 18 Kohlenstoffatomen, m eine ganze Zahl von 2 bis 6 und HX eine anorganische und/oder organische Säure darstellen, in einem Verhältnis dieser drei Komponenten von 5:1:1 bis 1:1:5.

Die erfindungsgemäß einzusetzenden mikrobiziden Mittel und ihre Wirkung werden nachfolgenden näher erläutert.

Die erfindungsgemäss einzusetzenden mikrobiziden Zubereitungen können beispielsweise die nachstehenden, in Tabelle 1 wiedergegebenen Zusammensetzungen aufweisen, die je nach Einsatzbereich gegebenenfalls auch noch weiter verdünnt werden können.

TABELLE 1

| Chemischer Stoff | Zubereitung (Mengenangaben entsprechen Gew.-%) | | | | | | | |
|---|---|---|---|---|---|---|---|---|
| | 1 | 2 | 3 | 4 | 5 | 6 | 7 | 8 |
| Kokos-propylendiamin-guanidinium-diacetat | 10,0 | | 0,50 | | 5,0 | 0,33 | 0,25 | 1,0 |
| Kokos-propylendiamin-guanidinium-hydrochlorid | | 8,0 | | 0,05 | | | | |
| Kokos-dimethyldichlorbenzyl-ammoniumchlorid | 5,0 | 6,0 | 0,25 | 0,01 | 3,0 | 0,33 | 0,25 | 1,0 |
| Didecylmethyl-polyoxyethylen-ammoniumpropionat | 5,0 | 8,0 | 0,25 | 0,01 | 3,0 | 0,33 | 0,50 | 5,0 |
| Wasser | ad 100,0 | | | | | | | |

Die bakterizide und fungizide Wirkung der erfindungsgemässen Zubereitungen wurde entsprechend den "Richtlinien für die Prüfung und Bewertung chemischer Desinfektionsverfahren" der Deutschen Gesellschaft für Hygiene und Mikrobiologie (Stand: 01.01.1981) unter Berücksichtigung der Anforderungen für die Aufnahme in die VII. Desinfektionsmittel-Liste der DGHM (Stand: 01.02.1984) untersucht.

Um die antimikrobielle Leistung und den Synergismus der erfindungsgemässen Zubereitungen deutlich aufzuzeigen, wurden verschiedene Vergleichspräparationen (Tabelle 2) hergestellt und die Abtötungszeiten der zu untersuchenden wässrigen, mikrobiziden Lösungen in Abhängigkeit von der Konzentration mit Hilfe des qualitativen Suspensionstests (Tabelle 3) ermittelt. Wegen der zu fordernden exakten Vergleichbarkeit aller antimikrobiellen Abmischungen wurden die Versuchsbedingungen so gewählt, dass die Testreihe zeitgleich mit Keimpopulationen einer identischen Quelle unter gleichen Prüfbedingungen durchgeführt wurde. Für den Suspensionsversuch wurden folgende Testkeime benutzt:

| | | |
|---|---|---|
| Staphylococcus aureus | ATCC 6538 | $1,9x10^9$ Keime/ml |
| Pseudomonas aeruginosa | ATCC 15442 | $2,2x10^9$ Keime/ml |
| Candida albicans | ATCC 10231 | $1,5x10^8$ Keime/ml |

Als der geeignete Enthemmungsmittelzusatz zur Nährlösung (CSL, Caseinpepton-Sojabohnenpepton-Lösung) erwies sich die Kombination 3% Tween 80, 3% Saponin, 0,1% Histidin, 0,1% Cystein. Weitere Angaben zur Versuchsausführung sind der oben beschriebenen DGHM-Prüfrichtlinie 1.2.2. zu entnehmen.

TABELLE 2

(Mengenangaben entsprechen Gew.-%)

| Mikrobizide Verbindung | VM1 | VM2 | VM3 | VM4 | VM5 | VM6 | VM7 | VM8 | VM9 | VM10 | VM11 | VM12 |
|---|---|---|---|---|---|---|---|---|---|---|---|---|
| Kokos-propylendiamin-guanidinium-diacetat | 1,00 | | | 0,25 | 0,75 | | | | | | 0,25 | |
| Kokos-dimethyldichlorbenzyl-ammonium-chlorid | | 1,00 | | 0,15 | | 0,25 | | | | | | |
| Didecylmethyl-polyoxyethylen-ammoniumpropionat | | | 1,00 | | 0,25 | 0,25 | | | | | | |
| Oligohexaymethylenbiguanid-hydrochlorid | | | | | | 0,50 | 1,00 | | | | 0,50 | |
| Kokos-dimethylbenzyl-ammoniumchlorid | | | | 0,20 | | | | | 1,00 | | 0,15 | |
| Chlorhexidin-diglukonat | | | | 0,40 | | | | 1,00 | | | | 0,50 |
| Didecyldimethyl-ammoniumchlorid | | | | | | | | | | 1,00 | 0,10 | 0,50 |
| Wasser ad 100,0 | | | | | | | | | | | | |

TABELLE 3

| Bakterizide und fungizide Wirkung im qualitativen Suspensionsversuch | | | | | | | | | | | | |
|---|---|---|---|---|---|---|---|---|---|---|---|---|
| | Minimale mikrobizide Konzentration in Abhängigkeit von der Zeit (Zahlenangaben entsprechen der Endkonzentration in % einer Verdünnungsreihe) | | | | | | | | | | | |
| Mittel | Testkeim Einwirkzeit | | | | | | | | | | | |
| Beispiel /Vergleich | S. aureus 5' | 15' | 30' | 60' | P. aeruginosa 5' | 15' | 30' | 60' | C. albicans 5' | 15' | 30' | 60' |
| 3 | 0,25 | 0,10 | 0,10 | 0,05 | 0,50 | 0,50 | 0,25 | 0,10 | 0,25 | 0,25 | 0,10 | 0,10 |
| 6 | 0,25 | 0,10 | 0,10 | 0,05 | 0,50 | 0,50 | 0,25 | 0,10 | 0,25 | 0,10 | 0,10 | 0,10 |
| 7 | 0,50 | 0,25 | 0,10 | 0,10 | 0,50 | 0,50 | 0,25 | 0,25 | 0,10 | 0,10 | 0,10 | 0,05 |
| VM 1 | 2,5 | 0,50 | 0,50 | 0,25 | 1,0 | 0,50 | 0,50 | 0,50 | 2,5 | 1,0 | 1,0 | 0,50 |
| VM 2 | 0,50 | 0,50 | 0,25 | 0,25 | 2,5 | 1,0 | 1,0 | 0,75 | 0,50 | 0,50 | 0,50 | 0,50 |
| VM 3 | 0,50 | 0,25 | 0,10 | 0,10 | 2,5 | 2,5 | 1,0 | 0,75 | 0,50 | 0,50 | 0,25 | 0,25 |
| VM 4 | 1,0 | 0,50 | 0,50 | 0,50 | 2,5 | 1,0 | 1,0 | 1,0 | 2,5 | 2,5 | 2,5 | 1,0 |
| VM 5 | 0,50 | 0,50 | 0,25 | 0,25 | 1,0 | 1,0 | 1,0 | 0,75 | 5,0 | 2,5 | 2,5 | 1,0 |
| VM 6 | 0,50 | 0,50 | 0,50 | 0,25 | 1,0 | 0,50 | 0,25 | 0,25 | 0,50 | 0,50 | 0,50 | 0,50 |
| VM 7 | > 5,0 | > 5,0 | 1,0 | 0,50 | 5,0 | 5,0 | 1,0 | 0,50 | > 5,0 | 5,0 | 1,0 | 0,50 |
| VM 8 | > 5,0 | 5,0 | 2,5 | 1,0 | 5,0 | 5,0 | 2,5 | 2,5 | 5,0 | 1,0 | 1,0 | 1,0 |
| VM 9 | 0,50 | 0,50 | 0,25 | 0,25 | 5,0 | 2,5 | 2,5 | 2,5 | 1,0 | 0,50 | 0,50 | 0,50 |
| VM 10 | 0,50 | 0,25 | 0,25 | 0,10 | 5,0 | 2,5 | 2,5 | 1,0 | 0,50 | 0,50 | 0,50 | 0,50 |
| VM 11 | 1,0 | 0,50 | 0,50 | 0,25 | 5,0 | 1,0 | 0,50 | 0,50 | 1,0 | 1,0 | 0,50 | 0,50 |
| VM 12 | 2,5 | 1,0 | 1,0 | 0,50 | 5,0 | 2,5 | 2,5 | 1,0 | 1,0 | 0,50 | 0,50 | 0,50 |

Die Ergebnisse gemäß Tabelle 3 zeigen, dass durch den Einsatz der erfindungsgemässen Mittel eine deutliche Wirkungssteigerung gegenüber der alleinigen Verwendung von quartären Ammoniumsalzen bzw. von Biguanid- oder Diguanidin-Verbindungen einschliesslich von Abmischungen dieser biologisch-aktiven Stoffe erzielt werden kann. Insbesondere ist bemerkenswert, dass die Stoffkombinationen gemäss der Erfindung eine erheblich verbesserte Wirkung gegenüber gramnegativen Keimen - bekannt als "Pseudomonas-Schwäche" zahlreicher germizider Wirkstoffe und Desinfektionsmittel-Formulierungen - aufweisen. Insgesamt betrachtet, findet die ermittelte herausragende mikrobizide Wirksamkeit der erfindungsgemässen Abmischungen lediglich eine hinreichende Erklärung unter der Annahme, dass ein synergistischer Effekt eintritt.

Die Prüfung der erfindungsgemässen mikrobiziden Zubereitungen gegenüber Hepatitis-B-Viren (HBV) erfolgte im Antigen-Inaktivierungstest (vgl. Zbl. Bakt. Hyg. I Abt. Orig. B 176, 1 (1982). Die Ergebnisse sind in Tabelle 4 zusammengestellt.

TABELLE 4.1

Wirkung auf die Antigenität des HBsAg

(Zu einem Teil HBsAg-haltigem Serum wurden 1 Teil Aqua bidest. bzw. 1 Teil 2 %iges Serumalbumin bzw. 1 Teil fetales Kälberserum und 8 Teile der 1,25-fachen Prüfkonzentration der mikrobiziden Zubereitung gegeben.)

| Mittel | Konzentration in % 1) | Einwirkzeit in Minuten | Cpm im HBsAg-Test nach Beendigung der Einwirkzeit: mit 1 Teil Aqua bidest. | mit 1 Teil 2 %igem Serumalbumin | mit 1 Teil fetalem Kälberserum |
|---|---|---|---|---|---|
| Beispiel 1 | 5 %ig | 0 (Antigenkontrolle ohne mikrobizide Zubereitung) | 6032 (100 %) | 5651 (100 %) | 5323 (100 %) |
| | | 5 | 114 (negativ) | 98 (negativ) | 82 (negativ) |
| | | 15 | 95 (negativ) | 93 (negativ) | 93 (negativ) |
| | | 60 | 119 (negativ) | 96 (negativ) | 111 (negativ) |
| | | 120 | 70 (negativ) | 93 (negativ) | 89 (negativ) |
| | | Mikrobizide Zubereitung ohne HBsAg | | 108 (0 %)* | |
| Beispiel 1 | 1 %ig | 0 (Antigenkontrolle ohne mikrobizide Zubereitung) | 6032 (100 %) | 5651 (100 %) | 5323 (100 %) |
| | | 5 | 119 (negativ) | 115 (negativ) | 249 (negativ) |
| | | 15 | 101 (negativ) | 101 (negativ) | 150 (negativ) |
| | | 60 | 110 (negativ) | 124 (negativ) | 77 (negativ) |
| | | 120 | 81 (negativ) | 82 (negativ) | 116 (negativ) |
| | | Mikrobizide Zubereitung ohne HBsAg | | 166 (0 %)* | |

TABELLE 4.2

| Mittel | Konzentration in % 1) | Einwirkzeit in Minuten | Cpm im HbsAg-Test nach Beendigung der Einwirkzeit mit 1 Teil Aqua bidest. | | mit 1 Teil 2 %igem Serumalbumin | | mit 1 Teil fetalem Kälberserum | |
|---|---|---|---|---|---|---|---|---|
| Beispiel 6 | 20 %ig | 0 (Antigenkontrolle ohne mikrobizide Zubereitung) | 6032 | (100 %) | 5651 | (100 %) | 5323 | (100 %) |
| | | 5 | 89 | (negativ) | 176 | (negativ) | 118 | (negativ) |
| | | 15 | 104 | (negativ) | 119 | (negativ) | 158 | (negativ) |
| | | 60 | 111 | (negativ) | 98 | (negativ) | 97 | (negativ) |
| | | 120 | 82 | (negativ) | 102 | (negativ) | 107 | (negativ) |
| | | Mikrobizide Zubereitung ohne HBsAg | | | 139 | (0 %)* | | |

1) bezogen auf die Zusammensetzung gemäß Tabelle 1.

* Die Nachweisgrenze des HBsAg im Ausria-II-Test beträgt laut Herstellerangabe das 2,1-fache der Cpm der negativen Kontrolle, - hier 227 Cpm, 349 Cpm bzw. 292 Cpm -

Den Prüfergebnissen der Tabelle 4 ist zu entnehmen, dass durch Einwirkung der mit Wasser hochverdünnten erfindungsgemässen Mittel in nur 5 Minuten selbst bei hoher Eiweissbelastung (Kälberserum) eine vollständige Zerstörung der immunologischen Reaktivität des HBsAg erreicht wird. Einem chemischen Desinfektionsmittel wird aufgrund der Prüfkriterien im Antigen-Inaktivierungstest eine HBV-inaktivierende Wirkung attestiert, wenn unter Einwirkung des Desinfiziens eine komplette Zerstörung

der immunologischen Reaktivität des HBsAg nachzuweisen ist. Die 5-Minuten-Einwirkzeit für verdünnte wässrige Lösungen der erfindungsgemässen Biozid-Kombinationen ist die kürzeste bei den Prüfungen verwendete Zeit und führte dennoch zu einer völligen Zerstörung des HBsAg. Ohne die tatsächlich mögliche minimale Einsatzkonzentration bereits festgestellt zu haben, ist den erfindungsgemässen mikrobiziden Zubereitungen deshalb eine ausgezeichnete HBV-inaktivierende Wirksamkeit zuzuschreiben.

Die Wirksamkeit der erfindungsgemässen Zubereitungen gegen den eine AIDS-Infektion verursachenden Human Immunodeficiency Virus (HIV bzw. auch HTLV III oder LAV) im Vergleich zu 0,7%igem Formaldehyd ergab eine um eine Zehnerpotenz höhere Inaktivierung des HIV bei 1%iger Konzentration der erfindungsgemässen Zubereitung 1 in nur 5 Minuten. Für das verwendete Aldehyd tritt im ausgeführten Suspensionsversuch negativ der Umstand eines toxischen Effektes hinzu.

Die erfindungsgemässen Zubereitungen sind weder toxisch noch führen sie in praxisrelevanter Anwendung zu Reizungen der Haut oder Schleimhaut. Für die Zubereitung des Beispiels 1 wurde die akute orale Toxizität an der Ratte in Anlehnung an die "OECD Principles of Good Laboratory Practice" in Testing of Chemicals (Paris, 1982) und gemäss der OECD-Richtlinie 401 (Mai 1981), 407 (Mai 1981) sowie den EG-Richtlinien 84/449/EWG mit einer oralen $LD_{50}$ > 2076 mg/kg bestimmt.

Werden Dentalformkörper aus Silikon-, Acrylat-, Alginat-, Polyether-, Gips- oder Hydrokolloidmassen mit den vorstehend definierten mikrobiziden Mitteln behandelt, so ist überraschend keine Veränderung der Dimensionen oder Oberflächenbeschaffenheit der Formkörper oder des damit erstellten Gipsmodells festzustellen.

Das gleiche gilt für den Fall, daß bei der Herstellung von Gips- oder Alginat-Abdruckmassen die erfindungsgemäßen Zubereitung anstelle des zur Zubereitung nötigen Wassers eingesetzt wird.

Die Desinfektionswirkung der aus solchen Alginat-Abdruckmassen hergestellten Formkörper gegen äussere Kontamination wurde wie folgt überprüft:

Handelsübliches Alginat-Abformpulver wurde anstelle von Wasser mit der vom Hersteller vorgeschriebenen Flüssigkeitsmenge der erfindungsgemässen mikrobiziden Zubereitung nach Beispiel 3 bzw. der gleichen Menge weiter verdünnter Lösungen derselben 1 Minute intensiv vermischt, in den sterilen Deckel einer Rodac-Platte verfüllt und über 3 Minuten bei 23 °C abgebunden. Der nach dieser Abbindezeit entstandene feste Prüfkörper aus Alginat-Abformmaterial wird dann als Testprüfkörper benutzt. In gleicher Weise werden Alginat-Formmassen unter Verwendung von Wasser, wie bisher üblich, hergestellt, die in den weiteren Untersuchungen als Kontrollprüfkörper dienen.

Als Testkeime für die nachfolgende Untersuchung wurden verwendet:

| | | Keime/ml |
|---|---|---|
| a) DGHM-Prüfkeime | | |
| Staphylococcus aureus | ATCC 6538 | $1,9x10^9$ |
| Escherichia coli | ATCC 11229 | $1,7x10^9$ |
| Pseudomonas aeruginosa | ATCC 15442 | $2,2x10^9$ |
| Proteus mirabilis | ATCC 14153 | $2,3x10^9$ |
| Candida albicans | ATCC 10231 | $1,5x10^8$ |
| b) Mundhöhlenkeime | | |
| Neisseria sp. | (frisch isolierter Stamm) | $3,3x10^9$ |
| Streptococcus salivarius | " | $1,0x10^9$ |
| Streptococcus pyogenes | " | $1,3x10^9$ |
| Streptococcus mitis | " | $2,5x10^9$ |

Die Kontamination der Test- und Kontrollprüfkörper erfolgte anschliessend dadurch, dass diese einzeln in 30 ml, in einer sterilen Petrischale befindlichen, Prüfkeimaufschwemmung eingelegt werden und nach mehrmaligem Drehen und Wenden nach 5 Minuten Kontaminationszeit der Keimsuspension entnommen werden. Der Zeitpunkt der Herausnahme der Prüfkörper ist zugleich der Startpunkt für die Einwirkzeit (hier

also = 0'), wobei die Prüfkörper in sterilen Petrischalen schräg lagern und bei Raumtemperatur über 15, 30, 60, 120 und 240 Minuten belassen werden.

Nach der jeweiligen Einwirkungszeit werden zur Ermittlung der log RF die Testkeime mittels der Abschwemm-Methode zurückgewonnen. Für diesen Zweck werden die Prüfkörper einzeln in einen 200 ml-Behälter, der 100 ml Inaktivierungsflüssigkeit enthält, verbracht und 2 Minuten mit Hilfe der Schüttelmaschine abgeschwemmt. Danach wird die Keimzahl (KBE) in der Abschwemmflüssigkeit bestimmt. Zusätzlich werden davon 0,1 ml und 1 ml in mit der Inaktivierungskombination versehene CSL-Röhrchen verimpft und bei 37° C bebrütet.

Aus der Differenz der log KBE der Kontrollprüfkörper (Ansetzen mit Wasser) und der log KBE der Testprüfkörper (Verwendung von Mittel 3) errechnet sich der log RF-Wert.

Der signifikante 5-log-Stufen RF wird von den Testprüfkörpern sowohl bei den DGHM-Prüfkeimen als auch bei den Mundhöhlenkeimen binnen 30′ bei Einsatz der erfindungsgemässen mikrobiziden Zubereitung nach Beispiel 3 bei der dort angegebenen Konzentration, binnen 60′ bei Verwendung einer auf das doppelte Volumen verdünnten Lösung und binnen 240′ bei Verwendung einer auf das vierfache Volumen verdünnten Lösung erreicht.

Das Alginat-Abformmaterial weist also bei Einsatz des Mittels 3 der vorliegenden Erfindung die Eigenschaft auf, sich selbst zu desinfizieren, so dass hier von Auto-Desinfektion gesprochen werden kann.

Im Suspensionstest ausgeführte Versuche zum Nachweis der HBV (HIV)-Inaktivierung an wie oben beschrieben gefertigten Prüfkörpern aus Alginat-Abformmaterial hatten zum Ergebnis, dass die Test-Prüfkörper - Herstellung derselben durch Anmischen von Alginat mit der wässrigen Lösung des erfindungsgemässen Mittels 3 bzw. Verdünnungen von 1:2, 1:3 und 1:5 mit der jeweiligen Menge Wasser - im Vergleich zu den Kontroll-Prüfkörpern - hergestellt durch Anmischen von Alginat mit Wasser - innerhalb von 5 bis 120 Minuten Einwirkzeit mit und ohne Eiweissbelastung im HBsAg-Inaktivierungstest zu einer rapiden Zerstörung des HBsAg führen. Diese Versuche unter praxisnahen Bedingungen durch Nachweis der HBV-Inaktivierung stellen somit erneut die hohe antimikrobielle Leistungsfähigkeit der mikrobiziden Zubereitungen entsprechend der vorliegenden Erfindung unter Beweis.

Damit kann der Zahnmedizin erstmals überraschend ein Mittel mit bakterizider, fungizider und viruzider Wirksamkeit im Sinne auto-desinfizierender Eigenschaften für die bislang nicht durchführbare Desinfektion von Alginat-Abdruckmaterialien zur Verfügung gestellt werden.

Die Eigenschaften der mit den erfindungsgemässen Zubereitungen hergestellten Alginat-Abformmassen unterscheiden sich nicht nachteilig von den nach dem Stand der Technik hergestellten Alginat-Abformmaterialien. Die Abbindezeiten, das Verhalten gegen Druck und lineare Massänderungen sind mit den Formmassen nach dem Stand der Technik vergleichbar und unterscheiden sich davon nicht. Überraschend ist die Verarbeitbarkeit der mit denerfindungsgemässen Zubereitungen hergestellten Abformmaterialien jedoch deutlich besser, und die Verträglichkeit mit Gips, insbesondere die saubere Trennung vom Gipsgegenabdruck, ist erheblich verbessert. Daraus ergeben sich insgesamt signifikante Vorteile für die Anwendung der erfindungsgemässen Zubereitungen bei der Herstellung von Alginat-Abformmassen im dentalen Anwendungsbereich.

Die Wirkungsbreite der erfindungsgemäss einzusetzenden Zubereitungen kann gegebenenfalls durch die Zugabe von Wirkstoffen mit bekannten mikrobiziden, spermiziden oder viruziden Eigenschaften verbessert und erweitert werden. So kann beispielsweise zur Erweiterung des Wirkungsspektrums das spermizid wirkende Nonylphenoloxethylat, das 9 oder 10 Mol Ethylenoxid pro Mol Verbindung enthält, zugegeben werden. Auch übliche Hilfs- oder Zusatzstoffe, wie Tenside, Farbstoffe, Geschmacks- oder Riechstoffe, Alkohole, Komplexbildner, Viskositätsregulatoren, Antischaummittel, Emulgatoren, Hautschutzstoffe, Korrosionsinhibitoren, Kältestabilisatoren, Lösungsvermittler, pH-Wert-regulierende Mittel und dergleichen können je nach Einsatzgebiet, soweit Verträglichkeit besteht, ohne Nachteile den erfindungsgemässen Zubereitungen zugegeben werden.

**Patentansprüche**

**1.** Verwendung von mikrobiziden wässrigen Zubereitungen, die

a) quartäre Ammoniumsalze der allgemeinen Formeln

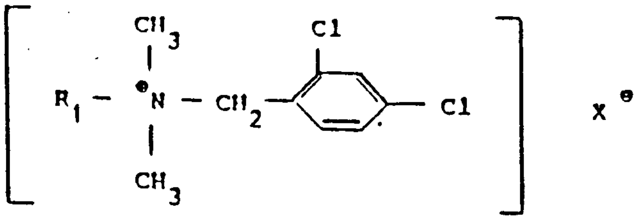

(I)

und

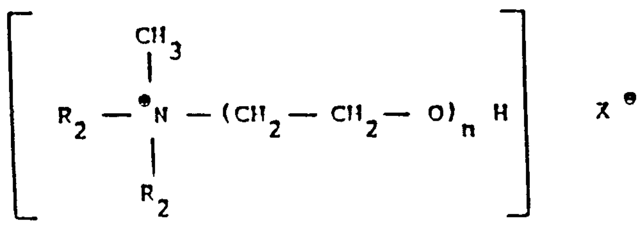

(II)

worin $R_1$ einen Alkylrest mit 8-18 Kohlenstoffatomen, $R_2$ einen Alkylrest mit geradzahliger Kohlenwasserstoffkette von 8 bis 16 C-Atomen, n eine Zahl von 1 bis 5 einschliesslich und $X^-$ das Anion einer anorganischen und/oder einer organischen Säure bedeuten, und

b) eine Diguanidin-Verbindung der allgemeinen Formel

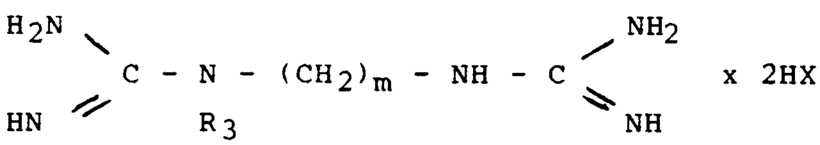

(III)

worin $R_3$ einen Alkylrest mit 8 bis 18 C-Atomen, m eine ganze Zahl von 2 bis 6 und HX eine anorganische und/oder organische Säure darstellen,

sowie gegebenenfalls übliche Zusatz- und Hilfsstoffe enthalten und und bei denen die Komponenten der Formeln I bis III im Verhältnis von 5:1:1 bis 1:1:5 vorliegen,

zur Desinfektion von Dentalformkörpern aus Acrylaten, Polyethern, Hydrokolloiden, Alginaten, Gips oder Silikonen.

2. Verwendung nach Anspruch 1, dadurch gekennzeichnet, dass die mikrobizide Zubereitung Nonylphenoloxethylat mit 9 oder 10 Mol Ethylenoxid pro Mol Verbindung enthält.

3. Verwendung der Zubereitungen nach Anspruch 1 oder 2 zur Desinfektion von Alginat- oder Gips-Abformmaterialien im Bereich der Zahnmedizin, dadurch gekennzeichnet, dass die mikrobiziden Zubereitungen bei der Herstellung der Abformmasse anstelle von Wasser eingesetzt werden.

4. Verwendung nach Anspruch 3, dadurch gekennzeichnet, dass die mikrobizide wässrige Zubereitung die Wirkstoffe in einer Gesamtkonzentration von höchstens 1% enthält.

## Claims

1. Use of microbicidal aqueous preparations which contain

a) quaternary ammonium salts of the general formula

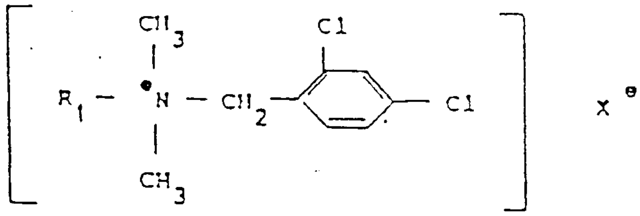

(I)

and

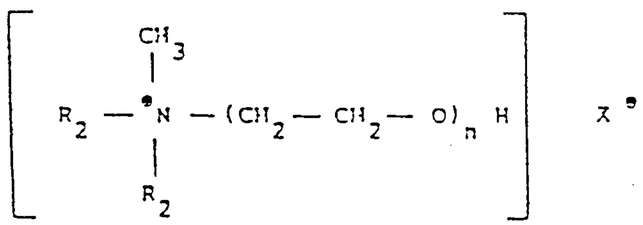

(II)

wherein $R_1$ represents an alkyl radical with 8-18 carbon atoms, $R_2$ represents an alkyl hydrocarbon chain with an even number, from 8 to 16, of carbon atoms, n is a number from 1 to 5 inclusive and $X^-$ is the anion of an inorganic and/or organic acid, and

b) a diguanidine compound of the general formula

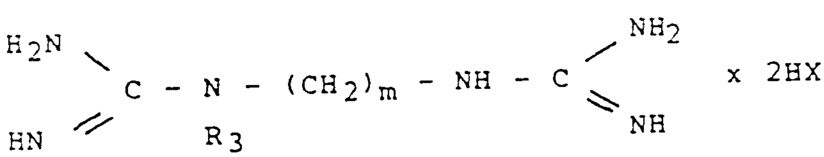

(III)

wherein $R_3$ represents an alkyl radical with 8 to 18 carbon atoms, m is an integer from 2 to 6 and HX is an inorganic and/or organic acid,

and optionally the usual additives and auxiliary substances and in which the components represented by formulas I to III are present in the ratio of 5:1:1 to 1:1:5,

to sterilise dentures made from acrylates, polyethers, hydrocolloids, alginates, plaster of Paris or silicones.

**2.** Use according to Claim 1, characterised in that the microbicidal preparation contains nonylphenoloxethylate with 9 or 10 moles of ethylene oxide per mole of compound.

**3.** Use of the preparations according to Claim 1 or 2 to sterilise alginate or plaster of Paris cast materials in the field of dental medicine, characterised in that the microbicidal preparations are used instead of water when preparing the cast items.

**4.** Use according to Claim 3, characterised in that the microbicidal aqueous preparations contain the active substance in a total concentration of at most 1%.

**Revendications**

1. Utilisation de préparations aqueuses microbicides contenant
   a) des sels d'ammonium quaternaire de formules générales

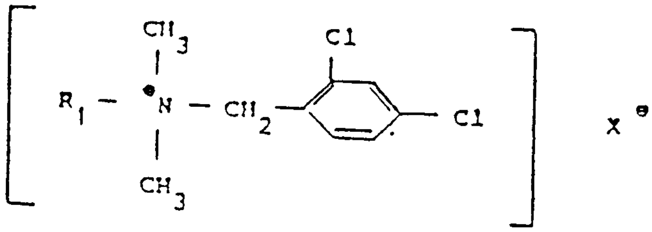

(I)

et

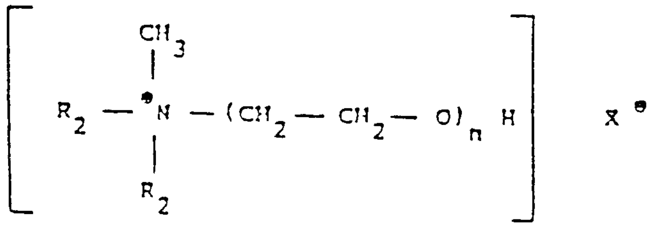

(II)

où $R_1$ désigne un radical alkyle possédant 8 à 18 atomes de carbone, $R_2$ un radical alkyle à chaîne d'hydrocarbure paire possédant 8 à 16 atomes de C, n un nombre entre 1 et 5 compris, et $X^-$ l'anion d'un acide inorganique et/ou d'un acide organique, et
b) un composé de diguanidine de formule générale

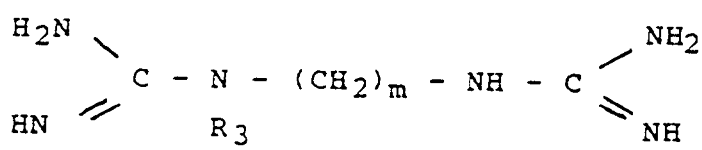

x 2HX (III)

où $R_3$ représente un radical alkyle possédant 8 à 18 atomes de C, m un nombre entier entre 2 et 6 et HX un acide inorganique et/ou organique,
ainsi que le cas échéant des additifs et adjuvants usuels et dans lesquelles les composants des formules I à III se trouvent dans le rapport de 5:1:1 à 1:1:5,
pour la désinfection de corps moulés dentaires en acrylates, polyéthers, hydrocolloïdes, alginates, plâtre ou silicones.

2. Utilisation selon la revendication 1, caractérisée en ce que la préparation microbicide contient de l'oxéthylate de nonylphénol comportant 9 à 10 moles d'oxyde d'éthylène par mole de composé.

3. Utilisation des préparations selon la revendication 1 ou 2 pour la désinfection de matériaux pour empreintes en alginate ou plâtre dans le secteur odontologique, caractérisée en ce que les préparations microbicides sont utilisées en remplacement de l'eau lors de la préparation de la masse pour empreintes.

4. Utilisation selon la revendication 3, caractérisée en ce que la préparation aqueuse microbicide contient les principes actifs en une concentration totale de 1 % au maximum.